# FASCICULE DE BREVET EUROPEEN

(11) **EP 2 736 552 B1**
(45) Date de publication et mention de la délivrance du brevet: **23.09.2015**
(21) Numéro de dépôt: 11757908.6
(22) Date de dépôt: 28.07.2011
(51) Int. Cl.: A61M 1/10

(54) **POMPE CARDIAQUE AMOVIBLE, ET PROCEDE MIS EN OEUVRE DANS UNE TELLE POMPE**
ABNEHMBARE HERZPUMPE UND VERFAHREN FÜR EINE SOLCHE PUMPE
REMOVABLE HEART PUMP, AND METHOD IMPLEMENTED IN SUCH A PUMP

(43) Date de publication de la demande: 04.06.2014
(73) Titulaire: Fineheart, 33600 Pessac (FR)
(72) Inventeur: GARRIGUE, Stéphane, F-33130 Bègles (FR)
(74) Mandataire: Kingolo, Alain
(86) Numéro de dépôt international: PCT/FR2011/051830
(87) Numéro de publication internationale: WO 2013/014339

(56) Documents cités:
- EP-A1- 1 129 736
- EP-A2- 0 764 448
- WO-A1-00/44417
- WO-A1-2004/101029
- WO-A1-2011/092394
- WO-A2-99/34847
- US-A- 5 507 629
- US-A- 5 888 241
- US-A- 6 135 729
- US-A1- 2006 036 127
- US-A1- 2008 004 485
- US-A1- 2011 184 224

## Description

La présente invention se rapporte à une pompe cardiaque artificielle permettant de réguler le débit sanguin, voir p.ex. WO2004/101029.

Le coeur est un muscle creux qui par sa contraction rythmique assure la progression du sang dans les vaisseaux. Il comporte quatre cavités. L'oreillette droite et l'oreillette gauche disposées dans la partie supérieure du coeur; le ventricule droit et le ventricule gauche disposés dans la partie basse.

Le ventricule droit est destiné à recevoir le sang en provenance de l'oreillette droite puis de l'éjecter dans l'artère pulmonaire. Cela constitue un « petit circuit» permettant d'envoyer le sang vers les poumons pour une réoxygénation.

Le ventricule gauche récupère le sang oxygéné depuis les poumons via l'oreillette gauche puis l'éjecte vers l'aorte pour apporter de l'oxygène à l'ensemble des tissus de l'organisme. Il s'agit de la « grande circulation » dite circulation systémique.

L'insuffisance cardiaque (IC), incapacité progressive du coeur à fournir un débit sanguin nécessaire aux besoins métaboliques d'un individu dans la vie courante, est la seconde cause de mortalité dans les pays occidentaux. Le traitement de l'insuffisance cardiaque, qui consiste à augmenter le débit sanguin de façon adaptée aux besoins du patient, est peu efficace avec les techniques actuelles, et coûte extrêmement cher.

On connaît le document US2009/0024212 décrivant une pompe pour traiter l'insuffisance cardiaque due à l'inactivité des valves sigmoïdes du coeur. Cette pompe présente une forme allongée s'étendant depuis l'intérieur du ventricule gauche jusqu'à l'intérieur de l'aorte de façon à remplacer la fonction des valves.

On connait également le document US6217541 décrivant une pompe cardiaque qui est également insérée à travers l'aorte jusqu'à l'intérieur du ventricule. L'extrémité de la pompe aspire le sang contenu dans le ventricule gauche puis le transfert vers l'aorte via un canal souple solidaire de l'extrémité de la pompe et disposé à travers les valves.

Les pompes décrites ci-dessus nécessitant une installation extrêmement complexe, et ne sont pas prévues pour une utilisation permanente.

On connaît aussi le document US6234772 décrivant une pompe rotative implantable. Cette pompe est de type à entrainement magnétique et permet de forcer la circulation du sang en évitant toute zone stagnante. Ce document reste muet quant à une quelconque mise en place efficace de la pompe.

Le document WO2010/010407 décrit une pompe rotative d'assistance cardiaque projetant le sang depuis le ventricule gauche à travers la valve aortique. Cette pompe est fixée à travers la valve aortique avec attaches de fixation dans l'aorte et au niveau de l'apex ventriculaire. Le moteur électrique est situé dans le conduit passant à travers la valve aortique.

On connaît enfin le document US2005/0107657 décrivant une pompe d'assistance ventriculaire gauche (pompe sanguine à flux mélangé) avec un circuit dit « radial » d'admission du sang et un circuit dit « axial » d'éjection du sang grâce à un propulseur rotatif situé au centre du dispositif. La base est maintenue au sein de la cavité ventriculaire gauche par une tige semi-rigide à travers l'apex du ventricule, alors que le sommet du dispositif passe à travers la valve aortique avec modification ou suppression fonctionnelle de cette valve. Chirurgicalement, une sternotomie avec mise en place d'une circulation extra-corporelle sont nécessaires, car il faut inciser au niveau de la racine de l'aorte. Ce document divulgue par ailleurs une équation d'efficience optimale entre le diamètre de la pompe et le nombre de tours/min du propulseur (jusqu'à 11 000 tours/min). Le diamètre de la pompe est donné à ∼ de 20-22 mm.

La présente invention a pour but une nouvelle pompe cardiaque peu complexe à installer par rapport à la mise en place de systèmes actuels.

Un autre but de l'invention est une simplicité dans la maintenance d'une telle pompe qui est prévue pour une utilisation à long terme.

L'invention a encore pour but une pompe peu invasive dans le ventricule du coeur et présentant une stabilité dans son maintien.

On atteint au moins l'un des objectifs précités avec une pompe cardiaque, telle que revendiquée, comprenant :
- un impulseur inséré dans le ventricule systémique d'un coeur, à travers la paroi de ce coeur, cet impulseur étant doté :
   - d'une membrane d'étanchéité et de fixation qui est en partie suturée sur la paroi externe du coeur de façon à solidariser l'impulseur avec la paroi du coeur,
   - d'un carter solidaire, directement ou indirectement, de la membrane d'étanchéité et de fixation, ce carter étant disposé à l'intérieur du ventricule systémique,
   - d'un moteur disposé dans le ventricule systémique et/ou dans l'épaisseur du ventricule, de façon à aspirer puis refouler le sang, depuis le fond, dans le ventricule systémique, à l'extérieur de l'impulseur et dans la direction de valves sigmoïdes du ventricule systémique, à travers le carter,
- une unité de gestion comprenant une alimentation et une unité de commande de l'impulseur; et
- une liaison filaire entre l'unité de gestion et l'impulseur.

En particulier, le moteur peut être est un moteur sans balai dit « brushless ».

Par ventricule systémique, on entend le ventricule dédié à la circulation sanguine pour alimenter le corps d'un patient en oxygène via l'aorte. En principe, ce rôle incombe au ventricule gauche, mais dans certaines situations pathologiques, ce rôle peut être joué par le ventricule droit.

Avec la pompe cardiaque selon l'invention, l'impulseur est solidement fixé à la paroi du coeur, le patient peut se déplacer activement sans risque de lésion. On agit directement sur le débit sanguin en contrôlant directement la circulation sanguine. La présente pompe s'adresse à tous les patients insuffisants cardiaques sans critères pré-requis.

Dans l'art antérieur tel que décrit dans les documents US2005/0107657 et WO2010/010407 notamment, le sang est directement propulsé dans l'aorte car la pompe traverse la valve aortique, ceci n'est pas le cas de la pompe de la présente invention. Dans l'art antérieur, un remplacement de la pompe nécessite une chirurgie très lourde car cela atteint la valve aortique.

L'impulseur peut avantageusement constituer un bloc amovible interchangeable via la paroi du ventricule systémique. Par ailleurs, la disposition et la forme de l'impulseur dans le ventricule systémique signifient que cet impulseur est entièrement accessible depuis l'extérieur du ventricule, et donc interchangeable, ce qui autorise une maintenance simplifiée sans geste chirurgical lourd de type sternotomie. Le remplacement est aisé en cas de panne ou d'usure.

Avantageusement, la membrane d'étanchéité et de fixation est fixée de façon à assurer une étanchéité totale et solidariser l'impulseur sur la partie basse du coeur à proximité de l'apex du coeur.

L'impulseur selon l'invention est un impulseur biocompatible de différents types, par exemple de type rotatif ou projectionnel.
De préférence, dans le premier cas, le moteur est de type rotatif et comprend un arbre d'entraînement de type rotor muni de pales ou de vis sans fin, cet arbre d'entrainement étant disposé dans le carter.

De préférence, le carter est un cylindre longiligne dont la paroi latérale est ajourée de façon à permettre l'écoulement de sang aspiré, et dont l'axe de révolution est en direction de valves sigmoïdes correspondantes. Une telle disposition permet d'éjecter le sang en direction de valves sigmoïdes, mais permet également d'aspirer efficacement le sang provenant de l'oreillette systémique. Par oreillette systémique, on entend l'oreillette associée au ventricule systémique.

Selon l'invention, l'unité de gestion peut être disposée à l'extérieur du patient, mais elle est de préférence en interne, et avantageusement dans la région épigastrique, dans la partie supérieure de l'abdomen. Ainsi, contrairement à des systèmes de l'art antérieur, l'alimentation selon l'invention est de préférence implantée dans son ensemble sans extériorisation. Pour ce faire, cette alimentation peut comprendre au moins une batterie, et de préférence une batterie rechargeable ; la recharge de la batterie pouvant éventuellement se faire par transduction percutanée.

La pompe selon l'invention peut ainsi être totalement implantée et autonome.

Selon une caractéristique avantageuse de l'invention, la pompe peut comprendre en outre une sonde, dite sonde d'activité pour recueillir l'activité cardiaque de façon à synchroniser le fonctionnement de l'impulseur avec l'activité électrosystolique cardiaque ; cette sonde d'activité peut être connectée à une paroi du coeur. Elle peut être reliée de façon filaire à l'unité de gestion. Cette configuration permet de synchroniser le fonctionnement de l'impulseur avec le rythme cardiaque.

Dans une configuration tout intégré, la sonde d'activité est reliée à l'unité de gestion via ladite liaison filaire. Dans ce cas, cette liaison filaire constitue la seule liaison entre l'unité de gestion et l'impulseur.

Selon un mode de réalisation avantageux de l'invention, la pompe cardiaque comprend une sonde de recueil d'activité cardiaque et de stimulation, dite sonde systémique, connectée à la paroi du ventricule systémique et apte à communiquer avec l'unité de gestion de façon filaire ou non, par télémétrie sans fil notamment. Cette sonde joue un double rôle de recueil de l'information cardiaque et de stimulation cardiaque pour contracter le muscle en réponse à une consigne provenant de l'unité gestion. Une seconde sonde de même type, dite sonde non systémique, peut être prévue connectée à la paroi du ventricule non systémique et apte à communiquer avec l'unité de gestion de façon filaire ou non, par télémétrie sans fil notamment. Dans ce cas, on peut commander ces deux sondes pour réaliser une stimulation bi-ventriculaire. Le fait de pouvoir stimuler le coeur permet d'associer une action directe de l'impulseur sur le débit sanguin et une action indirecte de contraction cardiaque. Le rythme cardiaque détecté par différentes sondes permet en outre de synchroniser le fonctionnement de l'impulseur avec l'activité cardiaque. En d'autres termes, l'impulseur est synchronisé sur l'activité systolique ventriculaire lorsqu'il est possible de recueillir une information sur l'activité cardiaque, ou peut fonctionner en mode continu.

On peut également envisager une autre sonde de recueil d'activité cardiaque et de stimulation, dite sonde oreillette, connectée à la paroi de l'oreillette systémique et apte à communiquer avec l'unité de gestion de façon à parfaire le système de recueil de l'activité cardiaque et de stimulation. La communication peut être filaire ou non, par télémétrie sans fil notamment.

Une sonde est autonome énergétiquement lorsqu'elle communique de façon sans fil avec l'unité de gestion.

En complément notamment de ce qui précède, la pompe selon l'invention peut avantageusement comprendre une sonde de recueil d'activité cardiaque, de stimulation et de défibrillation, dite sonde de défibrillation, connectée à la paroi du coeur et reliée de façon filaire à l'unité de gestion ; l'unité de commande étant en outre configurée en tant que défibrillateur.

On peut autrement prévoir une unité de gestion connectée de façon non filaire à un défibrillateur. Ce dernier peut être un défibrillateur externe (cutané) ou non, notamment automatique implantable indépendant mais communiquant avec l'unité de gestion par ondes électromagnétiques.

Selon un mode de réalisation avantageux de l'invention, on dispose en outre un second impulseur tel que décrit ci-dessus sur le ventricule non systémique et on le relie également à l'unité de gestion.

Dans le cadre d'une télémédecine, l'unité de gestion comprend un émetteur-récepteur sans fil pour transférer des données pour un suivi de télécardiologie. Ces données peuvent être des données hémodynamiques et ou rythmiques, mesurées par l'ensemble des sondes de la pompe.

Selon un autre aspect de l'invention, il est proposé un procédé pour réguler le débit sanguin dans un coeur au moyen d'une pompe cardiaque tel que décrit ci-dessus. Selon l'invention, on régule le débit sanguin en contrôlant la vitesse et la durée de fonctionnement de la pompe à partir de lois de commande prédéterminées ou à partir d'une consigne d'asservissement relative à l'activité cardiaque. Avec la consigne d'asservissement, on contrôle le débit sanguin en temps réel.

Avantageusement, on élabore la consigne d'asservissement en recueillant l'activité cardiaque au moyen d'une sonde connectée à la paroi du coeur et reliée de façon filaire à l'unité de gestion. On peut également réguler le débit sanguin en stimulant le coeur au moyen d'au moins une sonde de stimulation connectée à la paroi du coeur et reliée de façon filaire à l'unité de gestion.

D'autres avantages et caractéristiques de l'invention apparaîtront à l'examen de la description détaillée d'un mode de mise en oeuvre nullement limitatif, et des dessins annexés, sur lesquels :
- La figure 1 est une vue schématique simplifiée d'une pompe cardiaque selon l'invention insérée dans le ventricule gauche d'un coeur, et
- La figure 2 est une vue schématique d'une pompe cardiaque selon l'invention équipée d'une pluralité de sondes ou électrodes épicardiques de façon à synchroniser efficacement la pompe cardiaque par rapport à l'activité cardiaque.

Bien que l'invention n'y soit pas limitée, on va maintenant décrire une pompe cardiaque implantée dans le ventricule gauche d'un coeur qui est par principe le ventricule systémique. Mais, l'invention peut s'appliquer de la même manière à un ventricule droit lorsque ce dernier est le ventricule systémique.

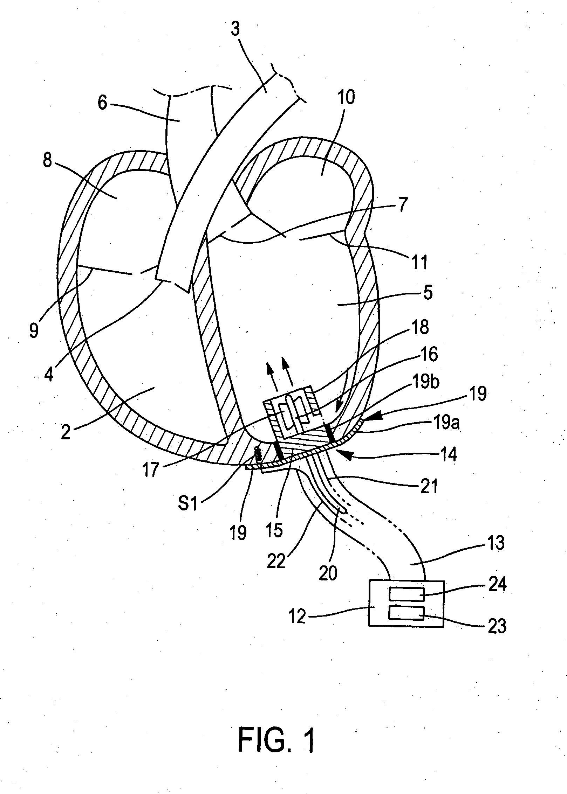

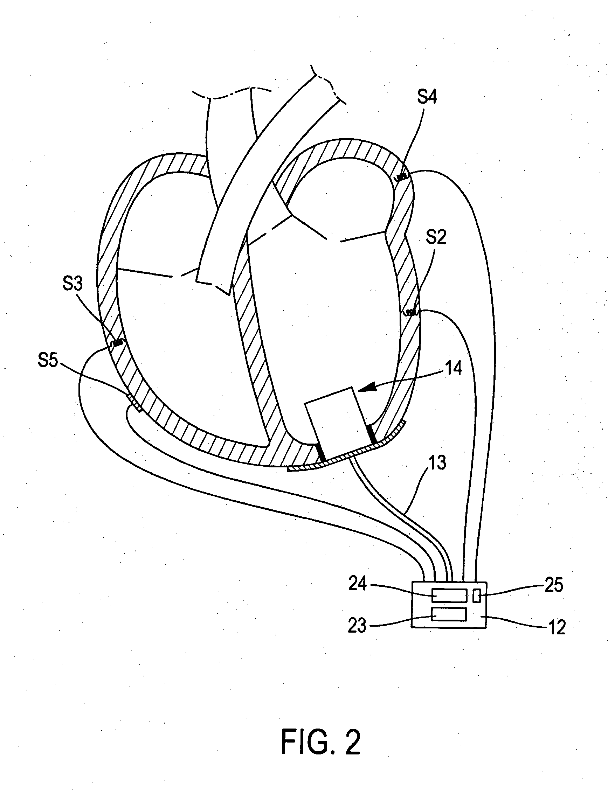

Sur les figures 1 et 2, les éléments communs aux diverses variantes ou formes de réalisation portent les mêmes références.

Sur les figures 1 et 2, le coeur est globalement désigné par la référence 1. On distingue le ventricule droit 2 qui a pour fonction d'éjecter le sang vers l'artère pulmonaire 3 à travers des valves sigmoïdes 4. Le ventricule gauche 5 a pour fonction de réaliser la circulation systémique en éjectant le sang rempli d'oxygène vers l'aorte 6 via des valves sigmoïdes 7.

L'oreillette droite 8 alimente le ventricule droit 2 en sang via des valves auriculo-pulmonaires 9. L'oreillette gauche 10 alimente le ventricule gauche 5 en sang via les valves mitrales 11.

La pompe selon l'invention comporte une unité de gestion 12 reliée par une liaison filaire 13 à un impulseur 14 inséré dans le ventricule gauche 5, au niveau de l'apex, c'est-à-dire à la pointe inférieure du ventricule gauche.

L'impulseur comporte un moteur de préférence de type brushless 15 placé à l'intérieur du ventricule gauche (ou ventricule systémique) de sorte qu'il est facilement accessible à la suite d'une mini-thoracotomie (incision chirurgicale) et ou d'une opération par voie épigastrique en comparaison d'une sternotomie où l'on ouvre complètement le thorax. Ce moteur peut être un moteur à entraînement magnétique équipé d'un rotor sous la forme d'un arbre d'entraînement 16. L'arbre peut être de type «vis sans fin» (« impeller ») permettant d'éjecter le sang depuis le fond du ventricule vers l'aorte 6. Cet arbre peut être aussi un arbre d'entraînement avec une hélice disposée à son extrémité libre. Cette hélice est conformée de telle sorte que la dynamique du fluide sanguin permet d'éjecter le sang vers l'aorte 6.

Un carter 18 de forme cylindrique est réalisé tout autour de l'arbre d'entraînement. Ce carter 18 comporte au moins une ouverture, de préférence plusieurs ouvertures en nid d'abeille par exemple, sur sa paroi latérale de façon à permettre l'aspiration de sang provenant de l'oreillette gauche et son évacuation par l'ouverture supérieure du cylindre formant carter 18 par l'action de l'hélice, d'une vis sans fin ou autres ... 17. L'axe de rotation du carter cylindrique 18 est dirigé vers l'orifice aortique. Une telle orientation est avantageusement obtenue lors de la mise en place de l'impulseur par suturage. L'homme du métier comprendra aisément que d'autres types de moteurs miniaturisés biocompatibles peuvent être utilisés pour aspirer et refouler le sang. D'une façon générale les matériaux utilisés pour la mise en oeuvre de la pompe selon l'invention sont biocompatibles et peuvent donc être implantés dans le corps du patient.

L'impulseur 14 est inséré dans l'apex du coeur et y est maintenu au moyen d'une membrane d'étanchéité et de fixation 19. D'autres types de membranes réalisant parfaitement l'étanchéité peuvent être envisagées. Cette membrane d'étanchéité et de fixation comprend une membrane d'étanchéité 19a associée, c'est-à-dire connectée directement ou non, à un système de fixation 19b, tel une collerette ou tout autre système. Le système de fixation 19b est fixé au moteur et/ou au carter dans l'épaisseur de la paroi du coeur. La membrane d'étanchéité 19a est de préférence suturée sur la paroi externe du coeur de façon à assurer une étanchéité totale entre le ventricule gauche 5 (ou ventricule systémique) et l'extérieur du coeur.

La membrane d'étanchéité et de fixation peut être de différente forme avec ou sans système de fixation disposé dans l'épaisseur de la paroi du coeur.

La liaison filaire 13 relie l'impulseur 14 à l'unité de gestion 12 qui comporte une alimentation 23 telle qu'une batterie et une unité de commande 24 configurable à distance. La liaison filaire 13 comporte une ligne de commande 21 permettant à l'unité de commande 24 d'envoyer des consignes de commande vers l'impulseur 14, la ligne de commande 21 pouvant être bidirectionnelle. Le câble 20 est un câble alimentation du moteur de l'impulseur 15. Le câble 22 permet de connecter électriquement l'unité de gestion 12 à une sonde d'activité S1 optionnelle insérée dans la paroi du coeur de façon à recueillir l'activité cardiaque du coeur. La sonde d'activité S1 peut être insérée à travers la membrane d'étanchéité et de fixation 19 ou bien au-delà afin de ne pas endommager l'étanchéité. Elle peut en outre être capable de stimuler le ventricule gauche ou droit. Dans ces cas, elle est disposée dans la paroi correspondant au ventricule gauche ou au ventricule droit.

Avec une telle pompe cardiaque selon l'invention, la liaison entre l'unité de gestion 12 et l'impulseur 14 est obtenue par l'unique liaison 13.

En fonctionnement, l'unité de gestion est configurée de façon à moduler la vitesse de rotation et la durée de fonctionnement du moteur en fonction de lois ou consignes de commande prédéterminées. Lorsqu'on dispose d'une sonde pour recueillir l'activité cardiaque comme par exemple la sonde d'activité S1, l'unité de commande 24 peut être configurée pour asservir le moteur au rythme cardiaque, en temps réel. Cet asservissement permet de synchroniser l'impulseur rotatif à la fréquence cardiaque.

De préférence, l'unité de gestion est implantée dans la région épigastrique, au sein de l'abdomen du patient. On peut ainsi prévoir que l'unité de commande 24 soit configurable à distance par communication sans fil.

Sur la figure 2, on voit un exemple de pompe cardiaque selon l'invention dans un mode de réalisation intégrant de nombreuses sondes ou électrodes épicardiques.

Les sondes disposées sur le coeur sont de types recueil d'information et stimulateur. Elles permettent d'identifier le début de l'activation électrique et de synchroniser l'impulseur sur l'ouverture des valves. Lorsque les deux ventricules sont soumis chacun à un impulseur, chaque impulseur est synchronisé à l'ouverture des valves correspondantes. Avantageusement, on adapte la fréquence de chaque impulseur de façon à délivrer de préférence un volume d'éjection systolique entre 20 et 35 ml pour chaque cycle cardiaque.

Étant donné que la mise en action d'un impulseur dans un ventricule à valve ouverte (durant la systole) augmente la quantité de sang éjectée, la pompe selon l'invention permet d'augmenter le volume d'éjection systolique et par conséquent, le débit sanguin.

Selon l'exemple illustré sur la figure 2, la pompe selon l'invention comporte une sonde de recueil d'activité cardiaque et de stimulation, dite sonde systémique S2 permettant notamment de stimuler le ventricule gauche par contraction musculaire. Cette sonde systémique S2, reliée à l'unité de gestion 12, est disposée dans la paroi du coeur au niveau du ventricule gauche. De la même manière une autre sonde de recueil d'activité cardiaque et de stimulation, dite sonde non systémique S3 est disposée sur la paroi du ventricule droit et est reliée à l'unité de gestion 12. Elle permet notamment de stimuler le ventricule droit par contraction musculaire. L'action combinée des deux sondes S2 et S3 permet de réaliser une stimulation bi-ventriculaire à partir de l'unité de commande 24 de façon à maintenir un rythme cardiaque selon une loi prédéterminée ou en réponse à des consignes données.

Sur la figure 2, on distingue également sur la paroi de l'oreillette gauche une sonde de recueil d'activité cardiaque et de stimulation, dite sonde oreillette S4, reliée à l'unité de gestion 12. Avantageusement, l'unité de commande 24 peut être configurée pour synchroniser la stimulation des sondes systémique S2 et non systémique S3 par rapport à l'information recueillie à partir de cette sonde S4.

En complément notamment de ce qui précède, chacune des sondes S2 et S4 peuvent jouer le rôle de la sonde d'activité S1.

Afin de traiter le risque de fibrillations ventriculaires, on prévoit au minimum un patch épicardique ou sonde de défibrillation S5 disposée sur la paroi externe du coeur, l'unité de commande étant configurée pour à la fois détecter une situation de fibrillation et de délivrer des chocs électriques de haute énergie.

Pour appréhender complètement l'activité cardiaque, on prévoit un capteur d'activité 25 du patient, tel qu'un accéléromètre ou un capteur de pression, disposé par exemple dans l'unité de gestion 12 ou intégré à l'une des sondes précitées. Un tel capteur peut être utile pour un patient atteint d'une insuffisance chronotrope afin de détecter et signaler à l'unité de commande une quelconque accélération de l'activité physique du patient.

On prévoit également un capteur hémodynamique pour détecter l'état hémodynamique du patient de façon à compléter les informations obtenues sur le rythme cardiaque et commander efficacement l'impulseur. Le capteur hémodynamique peut être un capteur d'accélération endocardique de type PEA ( « Peak Endocardial Acceleration » en langue anglaise ) implanté par exemple ensemble avec l'électrode S2.

La pompe cardiaque selon l'invention permet donc de réguler le débit sanguin afin d'éviter toute insuffisance cardiaque. En outre, elle peut être implantée dans le coeur par mini-thoracotomie. L'impulseur rotatif peut être inséré au niveau de l'apex (la pointe inférieure) du ventricule gauche et si nécessaire un second impulseur rotatif peut être inséré à l'apex du ventricule droit. Ces deux impulseurs peuvent avantageusement être connectés à une unité de gestion placée dans la région épigastrique. Il s'agit ainsi d'un système fermé sans extériorisation de matériel électrique et d'alimentation.

Bien sûr, l'invention n'est pas limitée aux exemples qui viennent d'être décrits et de nombreux aménagements peuvent être apportés à ces exemples sans sortir du cadre de l'invention.

## Revendications

1. Pompe cardiaque comprenant :
- un impulseur (14) destiné à être inséré dans le ventricule systémique (5) d'un coeur, à travers la paroi de ce coeur, cet impulseur étant doté :
- d'une membrane d'étanchéité et de fixation (19) destiné à être en partie suturée sur la paroi externe du coeur de façon à solidariser l'impulseur avec la paroi du coeur,
- d'un carter (18) solidaire de la membrane d'étanchéité et de fixation (19), ce carter étant destiné à être disposé à l'intérieur du ventricule systémique,
- d'un moteur (15) destiné à être disposé dans le ventricule systémique et/ou dans l'épaisseur du ventricule, de façon à aspirer puis refouler le sang, depuis le fond, dans le ventricule systémique, à l'extérieur de l'impulseur et dans la direction de valves sigmoïdes (7) du ventricule systémique, à travers le carter ; la membrane d'étanchéité étant associée à un système de fixation fixé, au moteur et/ou au carter, dans l'épaisseur de la paroi du coeur,
- une unité de gestion (12) comprenant une alimentation (23) et une unité de commande (24) de l'impulseur; et
- une liaison filaire (13) entre l'unité de gestion et l'impulseur.

2. Pompe cardiaque selon la revendication 1, **caractérisée en ce que** le moteur (15) est un moteur sans balai dit « brushless ».

3. Pompe cardiaque selon la revendication 1 ou 2, **caractérisée en ce que** l'impulseur constitue un bloc amovible interchangeable via la paroi du ventricule systémique.

4. Pompe cardiaque selon l'une quelconque des revendications précédentes, **caractérisée en ce que** la membrane d'étanchéité et de fixation (19) est fixée de façon à assurer une étanchéité totale et solidariser l'impulseur sur la partie basse du coeur à proximité de l'apex du coeur.

5. Pompe cardiaque selon l'une quelconque des revendications précédentes, **caractérisée en ce que** le moteur est de type rotatif et comprend un arbre d'entraînement de type rotor (16) muni de pales ou de vis sans fin, cet arbre d'entrainement étant disposé dans le carter.

6. Pompe cardiaque selon l'une quelconque des revendications précédentes, **caractérisée en ce que** le carter (18) est un cylindre longiligne dont la paroi latérale est ajourée de façon à permettre l'écoulement de sang aspiré, et dont l'axe de révolution est en direction de valves sigmoïdes correspondantes.

7. Pompe cardiaque selon l'une quelconque des revendications précédentes, **caractérisée en ce que** l'unité de gestion (12) est biocompatible de sorte qu'elle est apte à être disposée à l'intérieur du patient dans la région épigastrique.

8. Pompe cardiaque selon l'une quelconque des revendications précédentes, **caractérisée en ce que** l'alimentation (23) comprend au moins une batterie rechargeable.

9. Pompe cardiaque selon l'une quelconque des revendications précédentes, **caractérisée en ce qu'**elle comprend en outre une sonde, dite sonde d'activité (S1) pour recueillir l'activité cardiaque de façon à synchroniser le fonctionnement de l'impulseur avec l'activité électrosystolique cardiaque ; cette sonde d'activité étant connectée à une paroi du coeur.

10. Pompe cardiaque selon l'une quelconque des revendications précédentes, **caractérisée en ce qu'**elle comprend une sonde de recueil d'activité cardiaque et de stimulation, dite sonde systémique (S2), connectée à la paroi du ventricule systémique et apte à communiquer avec l'unité de gestion.

11. Pompe cardiaque selon l'une quelconque des revendications précédentes, **caractérisée en ce qu'**elle comprend en outre une sonde de recueil d'activité cardiaque et de stimulation, dite sonde non systémique (S3), connectée à la paroi du ventricule non systémique et apte à communiquer avec l'unité de gestion.

12. Pompe cardiaque selon l'une quelconque des revendications précédentes, **caractérisée en ce qu'**elle comprend en outre une sonde de recueil d'activité cardiaque et de stimulation, dite sonde oreillette (S4), connectée à la paroi de l'oreillette systémique et apte à communiquer avec l'unité de gestion.

13. Pompe cardiaque selon l'une quelconque des revendications précédentes, **caractérisée en ce qu'**elle comprend en outre une sonde de recueil d'activité cardiaque, de stimulation et de défibrillation, dite sonde de défibrillation (S5), connectée à la paroi du coeur et reliée de façon filaire à l'unité de gestion ; l'unité de commande étant en outre configurée en tant que défibrillateur.

14. Pompe cardiaque selon l'une quelconque des revendications 1 à 12, **caractérisée en ce que** l'unité de gestion (12) est connectée de façon non filaire à un défibrillateur.

15. Pompe cardiaque selon l'une quelconque des revendications précédentes, **caractérisée en ce qu'**elle comprend en outre un second impulseur disposé sur le ventricule non systémique et relié à ladite unité de gestion.

16. Pompe cardiaque selon l'une quelconque des revendications précédentes, **caractérisée en ce que** l'unité de gestion (12) comprend un émetteur-récepteur sans fil pour transmettre des données pour un suivi de télécardiologie.

## Patentansprüche

1. Herzpumpe, umfassend:
- einen Impulsgeber (14), der dazu bestimmt ist, in den systemischen Ventrikel (5) eines Herzens durch die Wand dieses Herzens eingesetzt zu werden, wobei dieser Impulsgeber ausgestattet ist:
- mit einer Dichtungs- und Befestigungsmembran (19), die dazu bestimmt ist, teilweise an der Außenwand des Herzens angenäht zu werden, um den Impulsgeber mit der Wand des Herzens fest zu verbinden,
- mit einem Gehäuse (18), das mit der Dichtungs- und Befestigungsmembran (19) fest verbunden ist, wobei dieses Gehäuse dazu bestimmt ist, innerhalb des systemischen Ventrikels angeordnet zu werden,
- mit einem Motor (15), der dazu bestimmt ist, in dem systemischen Ventrikel und/oder in der Dicke des Ventrikels angeordnet zu werden, um das Blut vom Boden anzusaugen, dann in den systemischen Ventrikel, außerhalb des Impulsgebers und in Richtung von Sigmoidklappen (7) des systemischen Ventrikels durch das Gehäuse zu fördern; wobei die Dichtungsmembran einem Befestigungssystem zugeordnet ist, das an dem Motor und/oder an dem Gehäuse in der Dicke der Wand des Herzens befestigt ist,
- eine Regelungseinheit (12), die eine Versorgung (23) und eine Einheit zur Steuerung (24) des Impulsgebers umfasst; und
- eine Kabelverbindung (13) zwischen der Regelungseinheit und dem Impulsgeber.

2. Herzpumpe nach Anspruch 1, **dadurch gekennzeichnet, dass** der Motor (15) ein bürstenloser, genannt "brushless", Motor ist.

3. Herzpumpe nach Anspruch 1 oder 2, **dadurch gekennzeichnet, dass** der Impulsgeber einen lösbaren Block bildet, der über die Wand des systemischen Ventrikels austauschbar ist.

4. Herzpumpe nach einem der vorhergehenden Ansprüche, **dadurch gekennzeichnet, dass** die Dichtungs- und Befestigungsmembran (19) derart befestigt ist, dass sie eine vollständige Dichtigkeit sicherstellt und den Impulsgeber an dem unteren Teil des Herzens in der Nähe der Herzspitze fest anbringt.

5. Herzpumpe nach einem der vorhergehenden Ansprüche, **dadurch gekennzeichnet, dass** der Motor vom rotierenden Typ ist und eine Antriebswelle vom Typ Rotor (16), die mit Blättern oder Schnecken versehen ist, umfasst, wobei diese Antriebswelle in dem Gehäuse angeordnet ist.

6. Herzpumpe nach einem der vorhergehenden Ansprüche, **dadurch gekennzeichnet, dass** das Gehäuse (18) ein langgestreckter Zylinder ist, dessen Seitenwand durchbrochen ist, um das Strömen von angesaugtem Blut zu ermöglichen, und dessen Rotationsachse in Richtung von entsprechenden Sigmoidklappen verläuft.

7. Herzpumpe nach einem der vorhergehenden Ansprüche, **dadurch gekennzeichnet, dass** die Regelungseinheit (12) biokompatibel ist, so dass sie geeignet ist, innerhalb des Patienten im epigastrischen Bereich angeordnet zu werden.

8. Herzpumpe nach einem der vorhergehenden Ansprüche, **dadurch gekennzeichnet, dass** die Versorgung (23) wenigstens eine wiederaufladbare Batterie umfasst.

9. Herzpumpe nach einem der vorhergehenden Ansprüche, **dadurch gekennzeichnet, dass** sie ferner eine Sonde, sogenannte Tätigkeitssonde (S1), umfasst, um die Herztätigkeit zu erfassen, um den Betrieb des Impulsgebers mit der elektrosystolischen Herztätigkeit zu synchronisieren; wobei diese Tätigkeitssonde an eine Wand des Herzens angeschlossen ist.

10. Herzpumpe nach einem der vorhergehenden Ansprüche, **dadurch gekennzeichnet, dass** sie eine Sonde zur Erfassung von Herztätigkeit und zur Stimulation, sogenannte systemische Sonde (S2), umfasst, die an die Wand des systemischen Ventrikels angeschlossen und geeignet ist, mit der Regelungseinheit zu kommunizieren.

11. Herzpumpe nach einem der vorhergehenden Ansprüche, **dadurch gekennzeichnet, dass** sie ferner eine Sonde zur Erfassung von Herztätigkeit und zur Stimulation, sogenannte nicht-systemische Sonde (S3), umfasst, die an die Wand des nicht-systemischen Ventrikels angeschlossen und geeignet ist, mit der Regelungseinheit zu kommunizieren.

12. Herzpumpe nach einem der vorhergehenden Ansprüche, **dadurch gekennzeichnet, dass** sie ferner eine Sonde zur Erfassung von Herztätigkeit und zur Stimulation, sogenannte Vorhofsonde (S4), umfasst, die an die Wand des systemischen Vorhofs angeschlossen und geeignet ist, mit der Regelungseinheit zu kommunizieren.

13. Herzpumpe nach einem der vorhergehenden Ansprüche, **dadurch gekennzeichnet, dass** sie ferner eine Sonde zur Erfassung von Herztätigkeit, zur Stimulation und zur Defibrillation, sogenannte Defibrillationssonde (S5), umfasst, die an die Wand des Herzens angeschlossen und mit der Regelungseinheit drahtverbunden ist; wobei die Steuerungseinheit ferner als Defibrillator ausgestaltet ist.

14. Herzpumpe nach einem der Ansprüche 1 bis 12, **dadurch gekennzeichnet, dass** die Regelungseinheit (12) mit einem Defibrillator drahtlos verbunden ist.

15. Herzpumpe nach einem der vorhergehenden Ansprüche, **dadurch gekennzeichnet, dass** sie ferner einen zweiten Impulsgeber, der an dem nicht-systemischen Ventrikel angeordnet und mit der Regelungseinheit verbunden ist, umfasst.

16. Herzpumpe nach einem der vorhergehenden Ansprüche, **dadurch gekennzeichnet, dass** die Regelungseinheit (12) einen drahtlosen Sender-Empfänger zur Übertragung von Daten für eine telekardiologische Betreuung umfasst.

## Claims

1. Heart pump comprising:
- an impeller (14) intended to be inserted in the systemic ventricle (5) of a heart, through the wall of said heart, this impeller being provided with:
- a sealing and fixing membrane (19) intended to be partly sutured on the external wall of the heart so as to make the impeller integral with the wall of the heart,
- a housing (18) integral with the sealing and fixing membrane (19), this housing being intended to be arranged within the systemic ventricle,
- a motor (15) intended to be arranged in the systemic ventricle and/or in the thickness of the ventricle, so as to aspirate and then eject blood, from the bottom, into the systemic ventricle, outside the impeller and in the direction of sigmoid valves (7) of the systemic ventricle, through the housing the sealing membrane being associated with a fixing system fixed, to the motor and/or the housing, within the thickness of the wall of the heart,
- a management unit (12) comprising a power supply (23) and a unit (24) for controlling the impeller; and
- a connecting wire (13) between the management unit and the impeller.

2. Heart pump according to claim 1, **characterized in that** the motor (15) is what is known as a brushless motor.

3. Heart pump according to claim 1 or 2, **characterized in that** the impeller constitutes a removable unit interchangeable via the wall of the systemic ventricle.

4. Heart pump according to any one of the preceding claims, **characterized in that** the sealing and fixing membrane (19) is fixed so as to ensure a perfect seal and make the impeller integral with the lower part of the heart near the cardiac apex.

5. Heart pump according to any one of the preceding claims, **characterized in that** the motor is of the rotary type and comprises a drive shaft of the rotor type (16) equipped with vanes or an endless screw, said drive shaft being arranged in the housing.

6. Heart pump according to any one of the preceding claims, **characterized in that** the housing (18) is a slender cylinder the side wall of which is of openwork construction so as to allow the flow of aspirated blood, and the axis of revolution of which is in the direction of corresponding sigmoid valves.

7. Heart pump according to any one of the preceding claims, **characterized in that** the management unit (12) is biocompatible so that it can be arranged inside the patient in the epigastric region.

8. Heart pump according to any one of the preceding claims, **characterized in that** the power supply (23) comprises at least one rechargeable battery.

9. Heart pump according to any one of the preceding claims, **characterized in that** it further comprises a sensor, known as an activity sensor (S1), for collecting data on cardiac activity so as to synchronize the operation of the impeller with the electrosystolic cardiac activity; this activity sensor being connected to a wall of the heart.

10. Heart pump according to any one of the preceding claims, **characterized in that** it comprises a sensor for collecting data on cardiac activity and for stimulation, known as a systemic sensor (S2), connected to the wall of the systemic ventricle and able to communicate with the management unit.

11. Heart pump according to any one of the preceding claims, **characterized in that** it further comprises a sensor for collecting data on cardiac activity and for stimulation, known as a non-systemic sensor (S3), connected to the wall of the non-systemic ventricle and able to communicate with the management unit.

12. Heart pump according to any one of the preceding claims, **characterized in that** it further comprises a sensor for collecting data on cardiac activity and for stimulation, known as an atrium sensor (S4), connected to the wall of the systemic atrium and able to communicate with the management unit.

13. Heart pump according to any one of the preceding claims, **characterized in that** it further comprises a sensor for collecting data on cardiac activity, for stimulation and for defibrillation, known as a defibrillation sensor (S5), connected to the wall of the heart and connected by wire to the management unit; the control unit being moreover configured as a defibrillator.

14. Heart pump according to any one of claims 1 to 12, **characterized in that** the management unit (12) is connected wirelessly to a defibrillator.

15. Heart pump according to any one of the preceding claims, **characterized in that** it further comprises a second impeller arranged on the non-systemic ventricle and connected to said management unit.

16. Heart pump according to any one of the preceding claims, **characterized in that** the management unit (12) comprises a wireless transmitter-receiver for transmitting data for monitoring by telecardiology.
